# EUROPÄISCHE PATENTANMELDUNG

(11) **EP 1 319 371 A2**
(43) Veröffentlichungstag der Anmeldung: **18.06.2003**
(21) Anmeldenummer: 02026645.8
(22) Anmeldetag: 29.11.2002
(51) Int. Cl.: A61B 17/12, A61F 5/00

(54) **Magenband**

(30) Priorität: 03.12.2001 DE 10158940
(71) Anmelder: Götz, Alois H., Dr., 91257 Pegnitz (DE)
(72) Erfinder: Götz, Alois H., Dr., 91257 Pegnitz (DE)
(74) Vertreter: Matschkur, Lindner Blaumeier Patent- und Rechtsanwälte

(57) **Zusammenfassung**

Magenband mit einem zu einem Ring formbaren, einen Hohlraum aufweisenden, mit einem Zuführungsschlauch verbindbaren oder verbundenen Schlingenabschnitt und einem Schließelement, dadurch gekennzeichnet, dass das Schließelement nahe dem Übergang zwischen dem Zuführungsschlauch und dem Schlingenabschnitt angeordnet ist, dessen anderes Ende zur Bildung des Rings mit dem Schließelement verbindbar ist.

## Beschreibung

Die Erfindung betrifft ein Magenband mit einem zu einem Ring formbaren, einen Hohlraum aufweisenden, mit einem Zuführungsschlauch verbindbaren oder verbundenen Schlingenabschnitt und einem Schließelement.

Magenbänder dieser Art werden zur Behandlung von Fettleibigkeit oder krankhafter Fettsucht eingesetzt. Der Schlingenabschnitt des Magenbands wird dabei operativ um den oberen Teil des Magens gelegt und durch Kopplung der Enden zu einer ringförmigen Schlinge geformt, so dass der Magen in einen kleineren, mit der Speiseröhre verbundenen Bereich oberhalb des Bands und einen größeren Bereich unterhalb des Bands geteilt wird. Nach dem Anlegen des Magenbands sind beide Bereiche lediglich über eine kleine Durchgangsöffnung miteinander verbunden, durch die die aufgenommene Nahrung passieren kann. Die Nahrung gelangt zunächst in den oberen Magenabschnitt, dessen Wand gedehnt wird. In der Folge stellt sich bei dem Patienten schnell ein Sättigungsgefühl ein, so dass er insgesamt weniger Nahrung zu sich nimmt, was zu einer Gewichtsreduktion führt.

Bei bekannten Magenbändern wird der Zuführungsschlauch mit einem Injektionsreservoir verbunden, das auch als Fluidport bezeichnet und unter der Haut fixiert wird. Nach der Operation kann die genaue Position des Injektionsreservoirs durch eine Röntgenkontrolle oder eine Ultraschalluntersuchung ermittelt werden. Im Rahmen der Therapie wird die Lage des Magenbands und dessen Innendurchmesser regelmäßig kontrolliert und gegebenenfalls angepasst. Zu diesem Zweck wird das Fluid wird mit einer Spritze in das Reservoir injiziert, was zu einer Verringerung des Innendurchmessers des aus einem elastischen Material bestehenden Schlingenabschnitts führt. Die innere, am Magen anliegende Seite des Magenbands weist eine geringere Wandstärke als die äußere Seite auf, die durch die Erhöhung der Fluidmenge elastisch gedehnt wird und den Innendurchmesser des Magenbands verkleinert, während der Außendurchmesser annähernd konstant bleibt. Umgekehrt kann der Ringinnendurchmesser bei Bedarf durch Entnahme des Fluids aus dem Injektionsreservoir wieder vergrößert werden.

Bei bekannten Magenbändern ist an einem Ende des Schlingenabschnitts eine Öse angeordnet, der Zuführungsschlauch befindet sich am entgegengesetzten Ende des Schlingenabschnitts. Zum Anlegen des Magenbands ist es daher erforderlich, den relativ langen Zuführungsschlauch durch die Öse zu ziehen, was sich in der Praxis aufgrund der beengten Platzverhältnisse als kompliziert und schwierig herausgestellt hat. Besonders problematisch sind Magenbänder, die aus einem harten, wenig flexiblen Material hergestellt sind. Das umständliche Einfädeln des Zuführungsschlauchs in den Verschluss erfordert vom Chirurgen große Geschicklichkeit, was in einer längeren Operationsdauer und dementsprechend erhöhten Kosten resultiert.

Der Erfindung liegt daher das Problem zugrunde, die genannten Nachteile zu vermeiden und ein Magenband zu schaffen, das einfacher angelegt werden kann.

Zur Lösung dieses Problems ist bei einem Magenband der eingangs genannten Art erfindungsgemäß vorgesehen, dass das Schließelement nahe dem Übergang zwischen dem Zuführungsschlauch und dem Schlingenabschnitt angeordnet ist, dessen anderes Ende zur Bildung des Rings mit dem Schließelement verbindbar ist.

Anders als bei bekannten Magenbändern werden lediglich die beiden Enden des Schlingenabschnitts durch das Schließelement miteinander verbunden, um die ringförmige Schlinge zu bilden. Da das Schließelement zwischen dem Zuführungsschlauch und dem Schlingenabschnitt liegt, erfolgt das Anlegen des Magenbands vollkommen unabhängig vom Zuführungsschlauch. Es ist nicht mehr erforderlich, den langen Zuführungsschlauch umständlich in das Schließelement einzufädeln und durchzuziehen, vielmehr verbindet der Operateur nur das andere Ende des Schlingenabschnitts mit dem Schließelement. Die dadurch erzielte Vereinfachung beim Bilden der Schlinge führt zu einer beträchtlichen Zeitverkürzung der Operation.

Gemäß einer ersten Weiterbildung des Erfindungsgedankens ist vorgesehen, dass das Schließelement ein erstes Rastelement umfasst, das mit einem am Ende des Schlingenabschnitts angeordneten zweiten Rastelement kraft- und/oder formschlüssig koppelbar ist. Beide Rastelemente werden derart miteinander verbunden, dass ein unbeabsichtigtes Lösen verhindert wird, wodurch sich eine besonders hohe Sicherheit ergibt. Die Verbindung erfolgt durch Einrasten, Einstecken oder Eindrücken.

Es ist vorteilhaft, ein Rastelement als Kugelzapfen auszubilden, der in eine hinterschnittene, vorzugsweise gegengleich ausgebildete Ausnehmung des anderen Rastelements einsteckbar ist. Grundsätzlich ist jedoch jede Form geeignet, die eine sichere Kopplung der beiden Rastelemente gewährleistet, anstelle eines Kugelzapfens kann daher ebenso ein Zylinder- oder Konuskörper verwendet werden, der in das andere Rastelement einsteckbar ist.

Gemäß einer zweiten Weiterbildung des Erfindungsgedankens ist vorgesehen, dass das Schließelement eine vorzugsweise als Öse ausgebildete Einstecköffnung umfasst, in die das andere Ende des Schlingenabschnitts einsteckbar ist. Diese Ausführung weist den Vorteil auf, dass anstelle des ganzen Zuführungsschlauchs lediglich das Ende des Schlingenabschnitts in die Öse gesteckt werden muss.

Die Öse ist im Bereich des Übergangs zwischen dem Zuführungsschlauch und dem Schlingenabschnitt angeordnet, sie liegt neben oder hinter den miteinander verbundenen Schlauchabschnitten des Schlingenabschnitts und des Zuführungsschlauchs ohne deren Funktion zu beeinträchtigen.

Es ist von Vorteil, wenn an dem anderen Ende des Schlingenabschnitts ein in die Öse einsteckbares Rastelement angeordnet ist, das nach dem Anlegen des Magenbands die Verbindung der beiden Enden des Schlingenabschnitts sichert und ein unbeabsichtigtes Lösen verhindert. Das Rastelement des erfindungsgemäßen Magenbands ist vorzugsweise in der Art eines Widerhakens ausgebildet, so dass eine Bewegung in Richtung der Einrastposition ermöglicht und in die entgegengesetzte Richtung verhindert wird.

Mit besonderem Vorteil ist an dem anderen Ende des Schlingenabschnitts ein vorzugsweise schnurartiger Einfädelabschnitt angeformt, der in die Öse eingeführt und durchgezogen wird, bis das Rastelement seine endgültige Position erreicht hat. Der Einfädelabschnitt kann vom Operateur mit einem Handhabungsinstrument gegriffen und durch die Öse gezogen werden, was auf einfache Weise möglich ist. Der Endabschnitt des Einfädelabschnitts kann einen geringeren Durchmesser als die Öse aufweisen, um das Einfädeln zu erleichtern.

In weiterer Ausgestaltung der Erfindung kann vorgesehen sein, dass der Einfädelabschnitt eine vorzugsweise als Querschnittsverringerung ausgebildete Sollbruchstelle aufweist. Nach dem Anlegen des Magenbands wird der überstehende Einfädelabschnitt nicht mehr benötigt, er kann daher nach dem Durchziehen durch die Öse abgetrennt werden. Die durch den verringerten Querschnitt gebildete Trennkerbe ist so bemessen, dass der Einfädelabschnitt beim Durchziehen durch die Öse nicht abreißt und anschließend durch eine erhöhte Kraft abgetrennt werden kann.

Zweckmäßig ist der Zuführungsschlauch des Magenbands mit einem Injektionsreservoir verbindbar oder fest verbunden, das bei der Operation im Körper des Patienten unterhalb der Haut fixiert wird. Um den Innendurchmesser des Magenbands zu verkleinern, wird die Fluidmenge erhöht, was zu einem größeren hydrostatischen Druck und in der Folge zu einer Dehnung der Innenseite des Magenbands führt. Die Erhöhung der Fluidmenge erfolgt durch eine Injektion durch die Haut des Patienten in das Injektionsreservoir.

Es ist zweckmäßig, wenn das Magenband aus einem strahlenundurchlässigen Material besteht, so dass der Innendurchmesser der Schlinge durch den Arzt kontrolliert werden kann, z.B. durch eine Röntgenaufnahme. Auf die gleiche Weise kann das Injektionsreservoir lokalisiert werden. Zusätzlich oder alternativ kann die Verwendung eines strahlenundurchlässigen Fluids vorgesehen sein.

Zur Herstellung des Magenbands ist biokompatibles Material, insbesondere Silikon, besonders geeignet.

Weitere Vorteile und Einzelheiten der Erfindung werden nachfolgend anhand eines besonders geeigneten Ausführungsbeispiels beschrieben. Die Figuren sind schematische Darstellungen und zeigen:
- Fig. 1: ein erfindungsgemäßes Magenband vor dem Anlegen in einer teilweise geschnittenen Ansicht;
- Fig. 2: das in Fig. 1 gezeigte Magenband nach dem Bilden der Schlinge; und
- Fig. 3: die Lage des Magenbands im Körper eines Patienten.

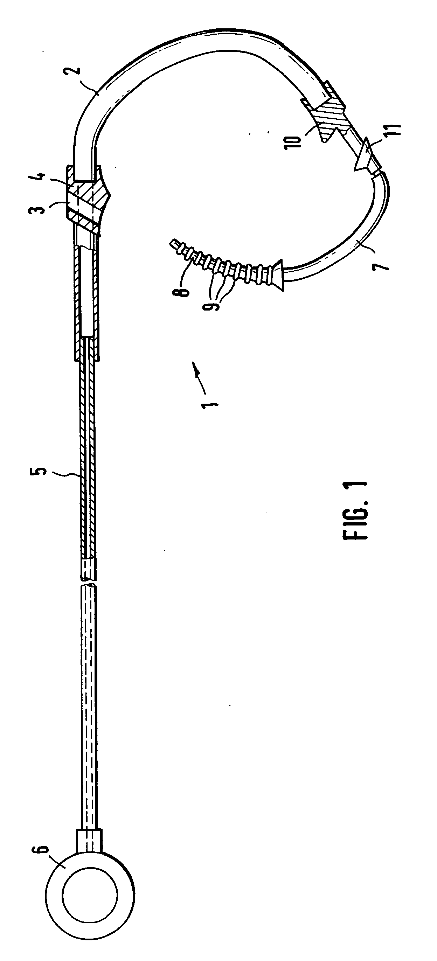

Das in Fig. 1 gezeigte Magenband 1 besteht im Wesentlichen aus einem schlauchartigen Schlingenabschnitt 2, der einerends über ein eine Öse 3 aufweisendes Schließelement 4 mit einem Zuführungsschlauch 5 verbunden ist. Am Ende des Zuführungsschlauchs 5 befindet sich ein Injektionsreservoir 6, das zum Injizieren oder zur Entnahme eines Fluids dient. Das Injektionsreservoir 6, der Zuführungsschlauch 5 und der Schlingenabschnitt 2 bilden einen zusammenhängenden rohrförmigen Aufnahmeraum für das Fluid. An dem Schlingenabschnitt 2 ist anderenends ein schnurartiger Einfädelabschnitt 7 angeformt, der aus Vollmaterial besteht. Die Einzelteile des Magenbands werden separat hergestellt und anschließend durch ein geeignetes Fügeverfahren miteinander verbunden.

Zum Anlegen des Magenbands 1 um den Magen eines Patienten wird der freie Endabschnitt 8 des Einfädelabschnitts 7 durch die Öse 3 gezogen. Um das Einfädeln zu erleichtern, weist der Endabschnitt 8 einen kleineren Durchmesser als die Öse 3 auf. Entlang des Endabschnitts 8 befinden sich ringförmige Querwülste 9, deren Durchmesser vom freien Ende in Richtung auf den Einfädelabschnitt 7 hin zunimmt. Die elastischen Querwülste 9 verhindern ein Herausrutschen des Endabschnitts 8 aus der Öse 3.

Zwischen dem Einfädelabschnitt 7 und dem Schlingenabschnitt 2 befindet sich ein Ansatz 10 mit einem in der Art eines Widerhakens ausgebildeten Rastelement 11. Das Rastelement 11 ist konusförmig ausgebildet, so dass es relativ leicht unter elastischer Verformung durch die Öse 3 hindurchgezogen werden kann und dort wieder seine Ursprungsform annimmt. Eine Bewegung in die entgegengesetzte Richtung ist jedoch nicht mehr möglich, da der Durchmesser der flachen Seite des Rastelements 11 größer als der Ösendurchmesser ist. Nach dem Verrasten sind die beiden Enden des Schlingenabschnitts 2 über den Ansatz 10 und das Schließelement 4 formschlüssig miteinander verbunden.

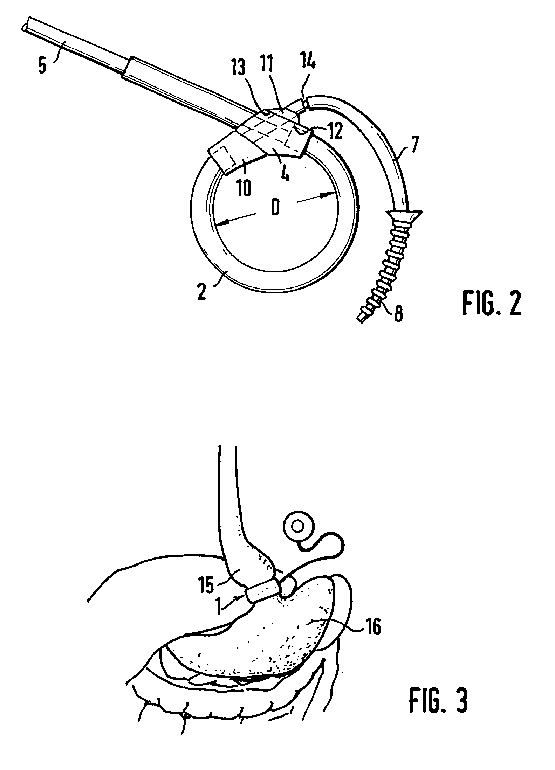

Fig. 2 zeigt das in Fig. 1 dargestellte Magenband 1 im angelegten Zustand, d.h. nach dem Bilden der Schlinge. Der Endabschnitt 8 des Einfädelabschnitts 7 und das Rastelement 11 sind in dieser Lage durch die Öse 3 hindurch gezogen. Wie in Fig. 2 zu erkennen ist, liegt die flache Seite 12 des Rastelements 11 formschlüssig an einer entsprechenden Gegenfläche 13 des Schließelements 4 an. Der von dem Schlingenabschnitt 2 und dem Zuführungsschlauch 5 gebildete Fluidraum befindet sich hinter dem Ansatz 10, der Verschlussmechanismus ist von dem Fluidraum getrennt und beeinträchtigt nicht dessen Funktion.

Zwischen dem Rastelement 11 und der Einfädelabschnitt 7 ist eine als Querschnittsverringerung ausgebildete Sollbruchstelle 14 angeordnet. Nach dem Anlegen des Magenbands 1 kann der Einfädelabschnitt 7 an dieser Trennkerbe abgetrennt werden. Anschließend wird der Fluidraum durch eine Injektion in das Injektionsreservoir 6 befüllt, woraufhin sich der Innendurchmesser D des Schlingenabschnitts 2 verringert, so dass die gewünschte Einschnürung des Magens des Patienten erfolgt.

Fig. 3 zeigt schematisch die Lage des Magenbands nach der Operation im Körper des Patienten. Wie dort zu erkennen ist, wird der Magen durch das Magenband 1 in einen kleineren, oberen Teil 15 und einen größeren, unteren Teil 16 geteilt.

Das Magenband 1 besteht aus einem biokompatiblen Material, insbesondere Silikon. Mit diesem Werkstoff können weiche und dennoch stabile Magenbänder hergestellt werden, die keine Druckkanten aufweisen. Es kann auch aus einem strahlenundurchlässigen Material bestehen, so dass die Position des Magenbands 1 und dessen Innendurchmesser D sowie die Lage des Injektionsreservoirs 6 durch eine Röntgenaufnahme kontrolliert werden können. Ebenso kann ein Röntgenkontrastmittel als Fluid verwendet werden.

## Patentansprüche

1. Magenband mit einem zu einem Ring formbaren, einen Hohlraum aufweisenden, mit einem Zuführungsschlauch verbindbaren oder verbundenen Schlingenabschnitt und einem Schließelement, **dadurch gekennzeichnet, dass** das Schließelement (4) nahe dem Übergang zwischen dem Zuführungsschlauch (5) und dem Schlingenabschnitt (2) angeordnet ist, dessen anderes Ende zur Bildung des Rings mit dem Schließelement (4) verbindbar ist.

2. Magenband nach Anspruch 1, **dadurch gekennzeichnet, dass** das Schließelement (4) ein erstes Rastelement umfasst, das mit einem am Ende des Schlingenabschnitts (2) angeordneten zweiten Rastelement kraft- und/oder formschlüssig koppelbar ist.

3. Magenband nach Anspruch 2, **dadurch gekennzeichnet, dass** ein Rastelement als Kugelzapfen ausgebildet ist, der in eine hinterschnittene, vorzugsweise gegengleich ausgebildete Ausnehmung des anderen Rastelements einsteckbar ist.

4. Magenband nach Anspruch 1 oder 2, **dadurch gekennzeichnet, dass** das Schließelement (4) eine vorzugsweise als Öse (3) ausgebildete Einstecköffnung umfasst, in die das andere Ende des Schlingenabschnitts (2) einsteckbar ist.

5. Magenband nach Anspruch 4, **dadurch gekennzeichnet, dass** an dem anderen Ende des Schlingenabschnitts (2) ein in die Öse (3) einsteckbares Rastelement (11) angeordnet ist.

6. Magenband nach Anspruch 5, **dadurch gekennzeichnet, dass** das Rastelement (11) als Widerhaken ausgebildet ist.

7. Magenband nach einem der vorangehenden Ansprüche, **dadurch gekennzeichnet, dass** an dem anderen Ende des Schlingenabschnitts (2) ein vorzugsweise schnurartiger Einfädelabschnitt (7) angeformt ist.

8. Magenband nach Anspruch 7, **dadurch gekennzeichnet, dass** der Einfädelabschnitt (7) eine vorzugsweise als Querschnittsverringerung ausgebildete Sollbruchstelle (14) aufweist.

9. Magenband nach Anspruch 7 oder 8, **dadurch gekennzeichnet, dass** der Einfädelabschnitt (7) einen Endabschnitt (8) mit einer Anzahl ringförmiger Querwülste (9) aufweist.

10. Magenband nach einem der vorangehenden Ansprüche, **dadurch gekennzeichnet, dass** der Zuführungsschlauch (5) mit einem Injektionsreservoir (6) zur Einstellung der Fluidmenge verbindbar oder verbunden ist.

11. Magenband nach einem der vorangehenden Ansprüche, **dadurch gekennzeichnet, dass** es aus einem strahlenundurchlässigen Material besteht und/oder mit einem strahlenundurchlässigen Fluid befüllbar ist.

12. Magenband nach einem der vorangehenden Ansprüche, **dadurch gekennzeichnet, dass** es aus einem biokompatiblen Material, insbesondere Silikon, besteht.
